## Europäisches Patentamt

## European Patent Office

## Office européen des brevets

(11) Publication number: **0 167 866**
**B1**

(12) **EUROPEAN PATENT SPECIFICATION**

(45) Date of publication of patent specification: **30.05.90**

(21) Application number: **85107261.1**

(22) Date of filing: **12.06.85**

(51) Int. Cl.⁵: **A 61 K 7/09**

(54) Process and composition for permanent waving.

(30) Priority: **13.06.84 US 620293**

(43) Date of publication of application:
**15.01.86 Bulletin 86/03**

(45) Publication of the grant of the patent:
**30.05.90 Bulletin 90/22**

(84) Designated Contracting States:
**CH DE FR GB IT LI**

(56) References cited:
**DE-A-3 048 124**
**DE-A-3 139 563**

**G.A. NOWAK, DIE KOSMETISCHEN PRäPARATE, 3rd ed., vol.2, 1984 Verlag für chemische Industrie H. Ziolowsky KG. Augsburg, 198**
**H. JANISTYN, HANDBUCH DER KOSMETIKA, 3rd ed., vol. 1, 1978, Dr. Alfred Hüthig Verlag Heidelberg, 197**

**The file contains technical information submitted after the application was filed and not included in this specification**

(73) Proprietor: **Revlon, Inc.**
**767 Fifth Avenue**
**New York, N.Y.10022 (US)**

(72) Inventor: **Ciaudelli, Joseph P.**
**54 Jean Street**
**Ramsey New Jersey (US)**
Inventor: **Goldberg, Marvin**
**4 Palomino Road**
**Marlboro New Jersey (US)**

(74) Representative: **Körber, Wolfhart, Dr. et al**
**Patentanwälte Dipl.-Ing. H. Mitscherlich Dipl.-Ing. K. Gunschmann Dr.rer.nat. W. Körber Dipl.Ing. J. Schmidt-Evers Dipl.-Ing. W. Melzer Steinsdorfstrasse 10**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a hair treating method and composition for permanent waving or straightening and more particularly relates to a two-step process for applying a cold permanent wave and neutralizer to hair and compositions for use in such process.

Hair is composed of keratin, and keratin is largely made up of relatively long-chain polyamides crosslinked by disulfide linkages.

It is well known that in the hair waving or straightening process the hair is first softened or relaxed, molded in the desired shape, and then rehardened in the new shape. The process involves subjecting the hair to the action of a reducing agent which relaxes the hair by rupturing the disulfide bonds in the keratin of the hair, followed by setting the hair into the desired configuration and then oxidizing or neutralizing the hair to recreate the disulfide bonds.

Over many years the reducing agents thioglycolates, such as ammonium thioglycolate, were preferred for their effectiveness to impart and hold the desired hair style. Notwithstanding their effectiveness as hair relaxers, thioglycolates were observed to cause irritation to scalp, damage to hair and undesirable odor during the hair treatment process.

More recently, the hair relaxing agents, sulfites and bisulfites have gained rapid success in both the cold permanent wave and hair straightener markets. The products containing sulfites and bisulfites as active ingredients lack the undesirable odor, offer the advantage of safety and low order of damage when used in the home-use market due to their longer processing time. However, these sulfites and bisulfites based relaxers are also not without problems. As a result of the reduction or rupturing of the disulfide bonds of hair keratin with a sulfite, the keratin molecule contains both the reduced (sulfhydryl-SH) and the oxidized (thiosulfite-SSO$_3$-) reaction products, the latter being known as a Bunte salt. The hair containing Bunte salt residues is referred to as Bunte salt hair. The neutralizing or rebuilding of the disulfide bonds ruptured by this process is difficult at best, since the Bunte salt residues are resistant to oxidation. Consequently, the unrestored disulfide bonds have a deleterious effect on the physical properties and appearance of the hair fibers, such as brittleness, weakened structure and loss of lustre.

It has now been surprisingly discovered that the drawbacks of sulfite-containing cold perm and relaxer formulations can be successfully overcome with a formulation and method of application the description of which follows.

In accordance with the present invention, a hair waving or straightening composition is provided which comprises an aqueous solution of the reducing agent, sodium metabisulfite, the swelling agent urea, the conditioning agent cetyl dimethyl (2-hydroxyethyl) ammonium phosphate and the buffering agent sodium borate decahydrate.

The composition when applied to hair relaxes or straightens the same by breaking the disulfide bonds of hair keratin and thereby rendering the hair soft and pliable. When a permanent configuration is desired, the soft, pliable hair is set into the desired configuration and a neutralizing preparation is applied thereto which quickly restores the disulfide bonds thereby re-establishing the strength of the hair and fixing the new configuration. The action of an oxidative agent, typically hydrogen peroxide, provides an immediate and effective result in reconstituting the disulfide bonds.

When the hair relaxer composition and the neutralizing preparation are sequentially employed as a cold wave permanent, a highly conditioned lusterous permanent wave results. This result is contrary to that obtained by the use of conventional sulfite-containing hair relaxer/neutralizer that tend to produce rubbery and brittle perms without adequate lustre.

While applicants do not intend to be bound by any theory, it is believed that urea as the swelling agent disrupts the hydrogen bonding in the hair shafts thereby exposing the disulfide bonds in hair keratin for greater penetration by the sulfite ions. It is also believed that the quaternary ammonium conditioning agent substantially blocks the formation of the Bunte salt moieties which therefore facilitates the reformation of the disulfide bonds during the neutralization step.

The hair relaxing or straightening composition of the present invention contains from about 5% to about 10% by weight and preferably 6% to 10% by weight of sodium metabisulfite, as the reducing agent, from about 5% to about 20% by weight and preferably 8% to 15% by weight of urea as hydrogen bond breaker for keratin and swelling agent, from about 0.2% to about 3.5% by weight of cetyl dimethyl (2-hydroxyethyl) ammonium phosphate as blocking agent against Bunte salt formation and about 5% to about 10% by weight sodium borate decahydrate as a buffering agent. The pH of the composition is about 6 to 7, and preferably 6.5 to 7.

While the above-described composition, having the specified ingredients, is the preferred formulation providing for superior hair relaxing qualities when singularly applied, and lusterous, permanent waves without Bunte salt residues when used in conjunction with a hydrogen peroxide neutralizer, the invention also encompasses the use of certain substitute ingredients in some formulations that provide satisfactory result. To wit: sodium sulfite, potassium sulfite and potassium metabisulfite may be used in place of sodium metabisulfite; cetyl dimethyl (2-hydroxyethyl) ammonium phosphate (sold under the tradename Bina Quat 44C by Ciba-Geigy Co.) may be replaced with other quaternary ammonium compounds such as stearyl-dimethyl-benzylammonium chloride, lauryl-dimethyl-benzylammonium bromide and cetyl-pyridinium chloride. The sodium borate decahydrate may be used alone or in combination with 2—4% by weight sodium carbonate, 3—6% by weight of sodium phosphate, 2—3% by weight sodium pyrophosphate and 3—4% by weight sodium citrate.

The composition can additionally include conventional additives, such as coloring and perfuming agents, emulsifiers, opacifiers and chelating agents. For example dyes, such as D & C Red 33, D & C Green 5 and D & C Yellow 10 were found to retain their color integrity in the hair relaxer formulations and as such could be used to distinguish between the curling or waving composition and the neutralizing formulation.

Contrary to the art recognized, concern that sulfite reduced hair oxidized with hydrogen peroxide tend to produce rubbery hair with waves that are limp and evanescent, it was surprising to obtain curls with the formulations of the present invention that were tight, lively, easy to comb in both wet and dry condition with no lack of lustre to hair.

The general formula and function of the ingredients used are as follows:

| Ingredients | Functions | % w/w |
|---|---|---|
| Sodium metabisulfite | (Reducing agent) | 5—10 |
| Sodium borate | (Buffering agent) | 5—10 |
| Urea | (Hydrogen bond breaker and hair swelling agent) | 5—20 |
| Ethylene diamine tetraacetic acid or derivatives | (Chelating agent) | 0—2.5 |
| Cetyl dimethyl (2-hydroxyethyl) ammonium phosphate solution | (Blocking agent for Bunte salt) | 0.2—3.5 |
| Sodium hydroxide (50% solution) | (pH adjuster) | 0.5—2.0 |
| Water | | q.s. to 100 |
| Coloring | | Trace |

The hair relaxer or straightener compositions of the present invention may be prepared by standard, art-recognized procedures. A convenient procedure involves dissolving in water at room temperature each of the ingredients by stirring, and then filtering to obtain a clear solution. The addition of solids produces endothermic heat as shown in the illustrative Examples 1 and 2.

Example 1

| Ingredients | Wt. (gm) | % w/w | pH | Temperature°C |
|---|---|---|---|---|
| Water | 648 | 64.8 | 5.42 | 17 |
| Sodium metabisulfite | 100 | 10.0 | 3.91 | 14.5 |
| Sodium borate | 100 | 10.9 | 5.98 | 11.0 |
| Urea | 100 | 10.0 | 6.15 | 7.0 |
| EDTA | 2 | 0.2 | 6.13 | 7.0 |
| Sodium hydroxide (50%) | 15 | 1.5 | 6.48 | 12.0 |
| Bina Quat 44C | 35 | 3.5 | 6.47 | 13.0 |
| Coloring | trace | | | |

## Example 2

| Ingredients | Wt. (gm) | % w/w | pH | Temperature°C |
|---|---|---|---|---|
| Water | 1974 | 65.8 | 6.41 | 21 |
| Sodium metabisulfite | 300 | 10.0 | 4.40 | 18 |
| Sodium borate | 300 | 10.0 | 6.30 | 14 |
| Urea | 300 | 10.0 | 6.61 | 9 |
| EDTA | 6 | 0.2 | 6.58 | 9 |
| Bina Quat 44C | 72 | 2.4 | 6.58 | 9 |
| Sodium hydroxide (50%) | 48 | 1.6 | 6.98 | 17 |

The following examples further illustrate the compositions of the present invention.

## Example 3

| Ingredients | Wt. (gm) |
|---|---|
| Water | 73.8 |
| Sodium metabisulfite | 7.5 |
| Sodium borate | 7.5 |
| Urea | 7.5 |
| EDTA | 0.2 |
| Bina Quat 44C | 2.6 |
| Sodium hydroxide (50%) | 0.9 |
| Resulting pH | 6.54 |

## Example 4

| Ingredients | Wt. (gm) | % w/w | pH |
|---|---|---|---|
| Water | 672.50 | 67.25 | 4.23 |
| Sodium metabisulfite | 100.00 | 10.00 | 4.02 |
| Sodium borate | 100.00 | 10.00 | 5.91 |
| Urea | 100.00 | 10.00 | 6.16 |
| Disodium copper (EDTA) | 2.50 | 0.25 | 6.17 |
| Bina Quat 44C | 5.00 | 0.50 | 6.18 |
| Sodium hydroxide (50%) | 13.25 | 1.325 | 6.51 |

## Example 5

| Ingredients | Wt. (gm) | % w/w | pH |
|---|---|---|---|
| Water | 69.2 | 69.2 | |
| Sodium metabisulfite | 10.0 | 10.0 | |
| Sodium borate | 5.0 | 5.0 | |
| Urea | 10.0 | 10.0 | |
| Sodium carbonate | 2.3 | 2.3 | |
| Bina Quat 44C | 3.5 | 3.5 | 6.55 |

## Example 6

| Ingredients | Wt. (gm) | % w/w | pH |
|---|---|---|---|
| Water | 68.5 | 68.5 | |
| Sodium metabisulfite | 10.0 | 10.0 | |
| Sodium borate | 5.0 | 5.0 | |
| Urea | 10.0 | 10.0 | |
| Sodium pyrophosphate | 3.0 | 3.0 | |
| Bina Quat 44C | 3.5 | 3.5 | 6.07 |

## Example 7

| Ingredients | % w/w |
|---|---|
| Water | 69.41 |
| Sodium metabisulfite | 10.00 |
| Sodium borate decahydrate | 10.00 |
| Urea | 10.00 |
| Cetyl dimethyl (2-hydroxyethyl) ammonium phosphate | 0.50 |
| Sodium hydroxide | 0.09 |
| | 100.00 |

The following examples show neutralizing solutions used in the two-step method of providing cold perm.

### Example 8

| Ingredients | % w/w |
|---|---|
| Water | 92 |
| Hydrogen peroxide (35%) | 8 |

### Example 9

| Ingredients | % w/w |
|---|---|
| Water | 95 |
| Hydrogen peroxide (35%) | 5 |

### Example 10

| Ingredients | % w/w |
|---|---|
| Water | 62.0 |
| Sodium bromate | 10.0 |
| Magnesium sulfate | 28.0 |
| pH | 4.15 |

The process according to the invention is suitable for relaxing or straightening kinky hair and for permanent waving of straight hair.

In hair relaxing or straightening, the hair is first cleaned, such as by shampooing, and thoroughly rinsed from the shampoo. Then the hair is patted with a towel to soak up excess moisture until the hair is just damp. The hair relaxer composition is then applied by saturating the hair with the composition using cotton balls. To insure complete saturation the hair may be combed until the hair is smooth and the composition is evenly distributed through the hair. The composition is then allowed to act for a period of time necessary for the softening of the hair, generally for about 15 to 60 minutes and then the hair is thoroughly rinsed and styled.

The permanent waving, in general, the cleaned and damp hair tresses are wound on curlers of suitable shape and dimensions. Then the tresses are impregnated with the hair relaxer composition. Alternatively, the curling on rollers may be accomplished after the hair is relaxed. Upon obtaining the desired relaxation, the hair is thoroughly rinsed with warm water followed by saturating the curls with the neutralizing solution. After a few minutes the rollers are removed, and the solution is evenly distributed by working the hair with the fingers, followed by thorough rinsing with water. The hair is then ready for styling and drying.

Compositions of the present invention were tested against commercial sulfite-containing products and were found to produce stronger curl pattern, longer lasting curls as well as superior appearance of lustre.

## Claims

1. A hair relaxing or straightening composition comprising by weight in an aqueous medium: from 5 to 10% of a reducing agent selected from sodium metabisulfite, sodium sulfite, potassium sulfite and potassium metabisulfite; from 5 to 20% of urea; from 0.2 to 3.5% of a quaternary ammonium compound selected from cetyl dimethyl (2-hydroxyethyl) ammonium phosphate, stearyl-dimethyl-benzylammonium chloride, lauryl-dimethyl-benzylammonium bromide and cetyl-pyridinium chloride; and from 5 to 10% of the buffering agent sodium borate decahydrate; said composition having a pH of between 6 and 7.

2. The composition of Claim 1 wherein said reducing agent is present in an amount of 6 to 10% by weight.

3. The composition of Claim 1 wherein said urea is present in an amount of 8 to 15% by weight.

4. The composition of Claim 1 wherein said buffering agent is present in combination with another buffering agent selected from 2 to 4% by weight of sodium carbonate; 3—6% by weight of sodium phosphate, 2—3% by weight of sodium pyrophosphate and 3—4% by weight of sodium citrate.

5. The composition of Claim 1 comprising by weight in an aqueous medium:

6 to 8% sodium metabisulfite;

8 to 15% of urea;

0.2 to 3.5% cetyl dimethyl (2-hydroxyethyl) ammonium phosphate; and

5 to 10% sodium borate decahydrate;
said composition having a pH of between 6.5 to 7.

6. A method of imparting a new configuration to the hair by using a hair relaxing or straightening composition of anyone of Claims 1 to 5 said method comprising:

a., first treating the hair with the hair relaxing composition of Claim 1;
b., setting the hair in the desired configuration;
c., rinsing the hair with water; and
d., treating said hair with an aqueous neutralizing solution containing from about 1% to about 10% by weight of hydrogen peroxide.

7. The method of Claim 6 wherein said configuration of hair is curled or straight.


## Patentansprüche

1. Haar-Entspannungs- und Streck-Mittel, dadurch gekennzeichnet, daß es in wässeriger Lösung—bezogen auf das Gewicht—enthält: 5 bis 10% einer reduzierenden Substanz ausgewählt aus Natriummetabisulfit, Natriumsulfit, Kaliumsulfit und Kaliummetabisulfit, von 5 bis 20% Harnstoff, von 0,2 bis 3,5% einer quaternären Ammoniumverbindung ausgewählt aus Cetyldimethyl-(2-hydroxyethyl)-ammoniumphosphat, Stearyldimethylbenzylammoniumbromid, Lauryldimethylbenzylammoniumbromid und Cetylpyridiniumchlorid sowie von 5 bis 10% der Puffersubstanz Natriumborat-Decahydrat und der pH-Wert zwischen 6 und 7 liegt.

2. Mittel gemäß Anspruch 1, dadurch gekennzeichnet, daß die reduzierende Substanz in einer Menge von 6 bis 10 Gew.-% vorliegt.

3. Mittel gemäß Anspruch 1, dadurch gekennzeichnet, daß der Harnstoff in einer Menge von 8 bis 15 Gew.-% vorliegt.

4. Mittel gemäß Anspruch 1, dadurch gekennzeichnet, daß die angeführte Puffersubstanz in Kombination mit einem anderen puffernden Stoff ausgewählt aus 2 bis 4 Gew.-% Natriumcarbonat, 3 bis 6 Gew.-% Natriumphosphat, 2 bis 3 Gew.-% Natriumpyrophosphat und 3 bis 4 Gew.-% Natriumcitrat anwesend ist.

5. Mittel gemäß Anspruch 1, dadurch gekennzeichnet, daß es in wässerigem Medium—bezogen auf das Gewicht—enthält:
6 bis 8% Natriummetabisulfit,
8 bis 15% Harnstoff,
0,2 bis 3,5% Cetyldimethyl(2-hydroxyethyl)ammoniumphosphat und
5 bis 10% Natriumborat.Decahydrat.

6. Verfahren zum Neufigurieren der Haare, dadurch gekennzeichnet, daß man ein Haarentspannungs- oder Streckungsmittel gemäß irgendeinem der Ansprüche 1 bis 5 verwendet und im Zuge des Verfahrens
a) zunächst das Haar mit dem Haarentsprannungsmittel entsprechend Anspruch 1 behandelt, dann
b) das Haar in die gewünschte Anordnung legt,
c) das Haar mit Wasser spült und
d) das Haar mit einer wässerigen Neutralisierungslösung mit einem Gehalt an etwa 1 bis etwa 10 Gew.-% Wasserstoffperoxid behandelt.

7. Verfahren gemäß Anspruch 6, dadurch gekennzeichnet, daß die gewünschte Anordnung der Haare gewellt oder glatt ist.


## Revendications

1. Composition de relaxation ou défrisage des cheveux comprenant, sur une base en poids, dans un milieu aqueux:
—de 5 à 10% d'un agent réducteur choisi parmi le métabisulfite de sodium, le sulfite de sodium, le sulfite de potassium et le métabisulfite de potassium;
—de 5 à 20% d'urée;
—de 0,2 à 3,5% d'un composé d'ammonium quaternaire choisi parmi le phosphate de cétyl diméthyl (hydroxy-2 éthyl) ammonium, le chlorure de stéaryl diméthyl benzyl ammonium, le bromure de lauryl diméthyl benzyl ammonium et le chlorure de cétyl pyridinium; et
—de 5 à 10% de l'agent tampon borate de sodium décahydraté;
ladite composition ayant un pH se situant entre 6 et 7.

2. Composition selon la revendication 1, dans laquelle ledit agent réducteur est présent en une quantité allant de 6 à 10% en poids.

3. Composition selon la revendication 1, dans laquelle ladite urée est présente en une quantité allant de 8 à 15% en poids.

4. Composition selon la revendication 1, dans laquelle ledit agent tampon est présent en combinaison avec un autre agent tampon choisi parmi 2 à 4% en poids de carbonate de sodium; 3—6% en poids de phosphate de sodium, 2—3% en poids de pyrophosphate de sodium et 3—4% en poids de citrate de sodium.

5. Composition selon la revendication 1, comprenant, sur une base en poids, dans un milieu aqueux:
—6 à 8% de métabisulfite de sodium;
—8 à 15% d'urée;
—0,2 à 3,5% de phosphate de cétyl diméthyl (hydroxy-2 éthyl) ammonium; et
—5 à 10% de borate de sodium décahydraté;
ladite composition ayant un pH se situant entre 6,5 et 7.

6. Procédé pour donner aux cheveux une nouvelle configuration par l'utilisation d'une composition de relaxation ou défrisage des cheveux telle que définie à l'une des revendications 1 à 5, ledit procédé consistant à:
a) traiter d'abord les cheveux avec la composition de relaxation des cheveux de la revendication 1;
b) placer les cheveux dans la configuration désirée;
c) rincer les cheveux à l'eau;
d) traiter lesdits cheveux par une solution aqueuse de neutralisation contenant d'environ 1% à environ 10% en poids de peroxyde d'hydrogène.

7. Procédé selon la revendication 6, dans lequel ladite configuration des cheveux est bouclée ou raide.